# EUROPEAN PATENT APPLICATION

(11) **EP 4 756 014 A1**
(43) Date of publication of application: **10.06.2026**
(21) Application number: 24849586.3
(22) Date of filing: 30.07.2024
(51) Int. Cl.: C12N 5/0786, C12N 15/85, C07K 16/28, C07K 14/705, C07K 14/725, C07K 14/73, A61K 39/00, A61P 35/00

(54) **RECOMBINANT IMMUNE CELL-BASED CELL THERAPEUTIC AGENT FOR TREATING CANCER VIA POLYCLONAL EXPANSION OF TUMOR-INFILTRATING T CELL IN TUMOR**

(30) Priority: 31.07.2023 KR 20230099732
(71) Applicant: Korea Advanced Institute of Science and Technology, Daejeon 34141 (KR)
(72) Inventor: PARK, Ji Ho, Daejeon 34141 (KR); YOON, Junyong, Daejeon 34141 (KR)
(74) Representative: Mewburn Ellis LLP
(86) International application number: PCT/KR2024/011170
(87) International publication number: WO 2025/029019

(57) **Abstract**

The invention relates to a recombinant immune cell into which (i) a first nucleic acid sequence encoding a single chain variable fragment (scFv) of an anti-CD3 antibody and (ii) a second nucleic acid sequence encoding a membrane protein are introduced. When the recombinant immune cells of the present invention are injected into tumors, the expression of the anti-CD3 epsilon chain scFv gene is induced on the cell membrane surface through membrane proteins, and intratumoral polyclonal proliferation of tumor-infiltrating T cells is induced through various costimulatory molecules of immune cells, thereby delaying or inhibiting tumor growth. In addition, the present invention relates to a composition comprising the recombinant immune cells as an active ingredient for treating cancer, wherein the recombinant immune cells are directly injected into a tumor.

## Description

### [Technical Field]

The present invention relates to a recombinant immune cell into which an anti-CD3 gene may be expressed on the cell membrane surface is introduced and a composition for treating cancer containing the same.

### [Background Art]

Many people all over the world are dying from various types of cancer and various methods including radiation and chemotherapy have been used to treat cancer over the past several years, but the number of cancer patients is still increasing and side effects of radiation and chemotherapy that cause illness and even lead to death in some patients are widely reported. In addition, in certain cancers, it is difficult to specify target antigens due to antigen heterogeneity, and in many cases, target antigens are not often widely known.

In particular, solid cancers, which mean abnormal tumors formed within tissues or organs, develop in various organs such as the lungs, breasts, colon, prostate, and brain. In general, it is difficult to specify cancer cell target antigens in solid cancers due to antigen heterogeneity. In addition, the types of cancer cell target antigens in solid cancers are often not widely known and tumors are often composed of various types of cells, thus the response to treatment varies in every case due to differences in the genetic and molecular characteristics of the cells, making it difficult to predict the treatment results.

In this regard, tumor-infiltrating T cell therapeutic agents that include isolating tumor-infiltrating T cells outside the body, proliferating the T cells and using the T cells as therapeutic agents, although the cancer cell target antigen is unknown, are currently being developed. The tumor-infiltrating T cell therapeutic agents have been reported to have a satisfactory therapeutic effect in a clinical trial targeting patients with metastatic melanoma, which is difficult to treat with conventional anticancer agents. However, the tumor-infiltrating T cell therapeutic agents have a problem in that the process of proliferating tumor-infiltrating T cells outside the body is very complicated and takes a long time, in relation to the method of mass-proliferating tumor-infiltrating T cells.

Therefore, with respect to the tumor-infiltrating T cell therapeutic agents, there is a demand and need for recombinant immune cells that are capable of simply and quickly proliferating tumor-infiltrating T cells inside the tumor, thus improving or enhancing the effects of conventional anticancer drugs to inhibit or delay tumor growth.

Therefore, as a result of research on methods to overcome the limitations of conventional tumor-infiltrating T cell therapeutic agents, the present inventors discovered recombinant immune cells that allow tumor-infiltrating T cells isolated from tumors to polyclonally proliferate directly within the tumors rather than outside the body, thereby exhibiting an excellent anticancer effect, and completed the present invention.

The information disclosed in this Background section is provided only for better understanding of the background of the present invention, and therefore it may not include information that forms the prior art that is already obvious to those skilled in the art.

### [Disclosure]

Therefore, the present invention has been made in view of the above problems, and it is one object of the present invention to provide a recombinant immune cell that enables tumor-infiltrating T cells to polyclonally proliferate directly within tumors for treatment of cancer, particularly solid tumors, and a composition for preventing or treating cancer containing the same.

It is another object of the present invention to provide the use of the recombinant immune cell for prevention or treatment of cancer.

It is another object of the present invention to provide the use of the recombinant immune cell for preparation of a composition for preventing or treating cancer.

It is another object of the present invention to provide a method of preventing or treating cancer using the recombinant immune cell.

### [Technical Solution]

In accordance with an aspect of the present invention, the above and other objects can be accomplished by the provision of a recombinant immune cell into which (i) a first nucleic acid sequence encoding a single chain variable fragment (scFv) of an anti-CD3 antibody and (ii) a second nucleic acid sequence encoding a membrane protein are introduced.

In accordance with another aspect of the present invention, there is provided a pharmaceutical composition for preventing or treating cancer containing the recombinant immune cell and a pharmaceutically acceptable carrier.

In accordance with another aspect of the present invention, there is provided the use of the recombinant immune cell for preventing or treating cancer.

In accordance with another aspect of the present invention, there is provided the use of the recombinant immune cell for preparation of a composition for preventing or treating cancer.

In accordance with another aspect of the present invention, there is provided a method of preventing or treating cancer including administering the recombinant immune cell.

### [Description of Drawings]

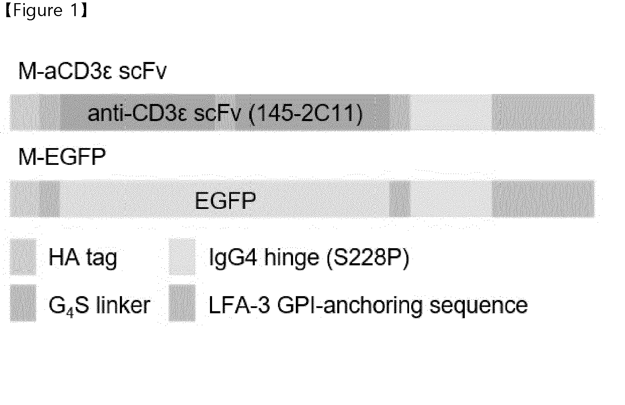

FIG. 1 is a schematic diagram illustrating a synthetic gene (M-aCD3εscFv; top of FIG. 1) obtained by combination of a variable region sequence of an anti-CD3ε antibody (145-2C11) with a GPI-anchoring sequence of a GPI-anchoring protein (LFA-3) and a synthetic gene (M-EGFP; bottom of FIG. 1) obtained by combination of a sequence encoding a fluorescent protein (EGFP) with the GPI-anchoring sequence of GPI-anchoring protein (LFA-3).

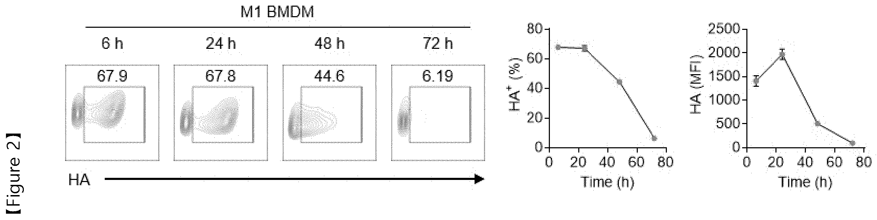

FIG. 2 shows the result of flow cytometry to validate the expression of M-aCD3εscFv cell membrane over time after transfection of proinflammatory bone marrow-derived macrophages (M1 BMDM) with M-aCD3εscFv mRNA.

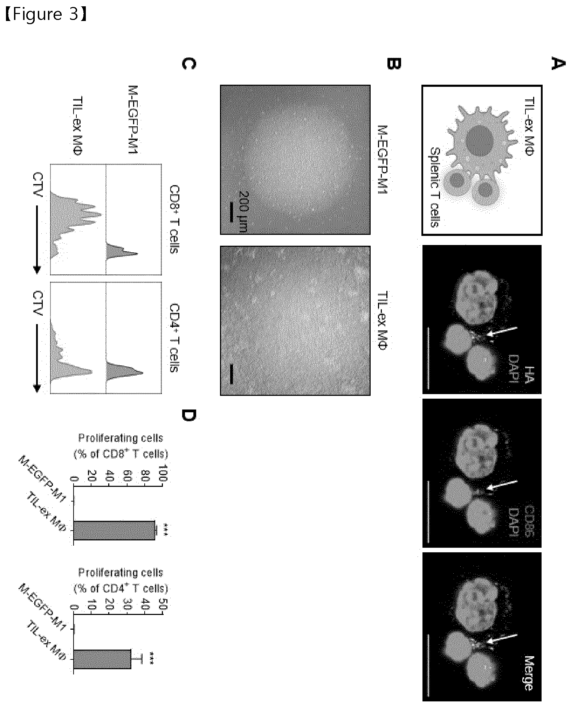

FIGs. 3A to 3D show the results of the interaction between TIL-ex MΦ and spleen T cells (FIG. 3A) and the results of spleen T cell proliferation (FIGs. 3B to 3D) after co-culture of T cells isolated from mouse spleen with M-EGFP-M1 or TIL-ex MΦ produced by transfecting M1 BMDM with M-EGFP or M-aCD3εscFv mRNA.

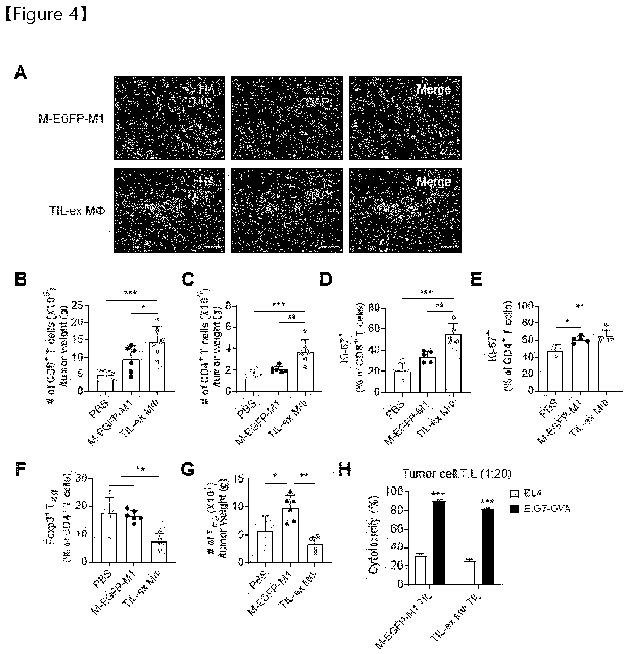

FIGs. 4A to 4H show the results of the interaction between intratumoral T cells (TILs) and macrophages (FIG. 4A), the number of CD8⁺ T cells and CD4+ T cells (FIGs. 4B and 4C), the proportion of Ki-67⁺ cells (FIGs. 4D and 4E), the results of validation of the proliferation of Foxp3⁺ Treg cells (FIGs. 4F and 4G), and the results of validation of the ability of TILs to kill tumor antigen-specific tumor cells (FIG. 4H) after intratumoral injection of TIL-ex MΦ or M-EGFP-M1 into a B16F10 mouse melanoma model.

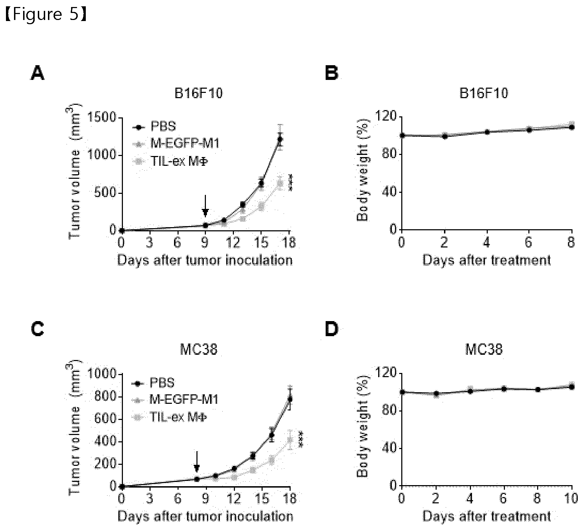

FIGs. 5A to 5D show the tumor suppression effect and whether or not changes in body weight occur after intratumoral injection of TIL-ex MΦ or M-EGFP-M1 into a B16F10 mouse melanoma model (FIGs. 5A and 5B), and the tumor suppression effect and whether or not changes in body weight occur after intratumoral injection of TIL-ex MΦ or M-EGFP-M1 into an MC38 mouse colon cancer model (FIGs. 5C and 5D).

### [Best Mode]

Unless defined otherwise, all technical and scientific terms used herein have the same meanings as those appreciated by those skilled in the field to which the present invention pertains. In general, the nomenclature used herein is well-known in the art and is ordinarily used.

In the present invention, with respect to a tumor-infiltrating T cell therapeutic agent for treating cancer, a recombinant immune cell was developed that can simply and rapidly proliferate tumor-infiltrating T cells within a tumor, improve or enhance the effect of conventional anticancer agents, and suppress or delay tumor growth. In addition, it was found that, when the immune cell of the present invention is injected into a tumor, tumor-infiltrating T cells are directly polyclonally proliferated *in vitro* and in the tumor through scFv including the epsilon chain of anti-CD3 antibody expressed on the cell membrane, thereby delaying or suppressing tumor growth and thus exhibiting an excellent anticancer effect.

As used herein, the term "T cell" refers to a lymphocyte that conducts antigen-specific adaptive immunity and the T cell is classified into a naive T cell that has not yet encountered an antigen, a mature T cell that has encountered an antigen, and a central memory T cell. The mature T cell includes cytotoxic T cells, αβT cells, and γδT cells, and the central memory T cell (T_{CM}) includes T cells having TCRαβ and TCRγδ.

In one aspect, the present invention is directed to a recombinant immune cell into which (i) a first nucleic acid sequence encoding a single chain variable fragment (scFv) of an anti-CD3 antibody and (ii) a second nucleic acid sequence encoding a membrane protein are introduced.

As used herein, the term "anti-CD3 antibody" encompasses both polyclonal antibodies and monoclonal antibodies and may have a whole antibody. The whole antibody has a structure having two full-length light chains and two full-length heavy chains, and has a structure including a constant region, and each light chain is linked to a heavy chain by a disulfide bond.

In the present invention, the whole antibody of the anti-CD3 antibody includes IgA, IgD, IgE, IgM, and IgG forms, and IgG includes IgG1, IgG2, IgG3, and IgG4 as subtypes.

As used herein, the term "anti-CD3 antibody" may be any known anti-CD3 antibody and may bind to human and mouse CD3.

In the present invention, the scFv of the anti-CD3 antibody may include a heavy chain variable region amino acid sequence of SEQ ID NO: 1 or SEQ ID NO: 5, and a light chain variable region amino acid sequence of SEQ ID NO: 3 or SEQ ID NO: 7, but is not limited thereto.

In the present invention, the first nucleic acid sequence encoding the scFv of the anti-CD3 antibody may include a sequence of SEQ ID NO: 2 or SEQ ID NO: 6, and a sequence of SEQ ID NO: 4 or SEQ ID NO: 8, but is not limited thereto.

In the present invention, the scFv of the anti-CD3 antibody may include an amino acid sequence of SEQ ID NO: 9, but is not limited thereto.

In the present invention, the first nucleic acid sequence may include a sequence of SEQ ID NO: 10, but is not limited thereto.

Meanwhile, the scFv of the anti-CD3 antibody may include a heavy chain variable region and a light chain variable region including a sequence having a sequence homology of 80% or more, preferably 90% or more, more preferably 99% or more, with the heavy chain variable region of SEQ ID NO: 1 or 5 and the light chain variable region of SEQ ID NO: 3 or 7, respectively, and a substituted antibody or scFv of the antibody having the same properties as the anti-CD3 antibody according to the present invention or the scFv of the anti-CD3 antibody falls also within the scope of the scFv of the anti-CD3 antibody according to the present invention.

In addition, the anti-CD3 antibody or scFv thereof according to the present invention also includes an antibody or scFv thereof in which a part of the amino acid sequence in the anti-CD3 antibody or scFv thereof according to the present invention is substituted by conservative substitution.

As used herein, the term "conservative substitution" refers to modification of a polypeptide including substitution of one or more amino acids with amino acids having similar biochemical properties without causing loss of the biological or biochemical function of the polypeptide. The term "conservative amino acid substitution" refers to substitution of amino acid residues with amino acid residues having similar side chains thereto. Classes of amino acid residues having similar side chains are defined and are well known in the art. These classes include amino acids having basic side chains (e.g. lysine, arginine, histidine), amino acids having acidic side chains (e.g. aspartic acid, glutamic acid), amino acids having uncharged polar side chains (e.g. glycine, asparagine, glutamine, serine, threonine, tyrosine, cysteine), amino acids having non-polar side chains (e.g., alanine, valine, leucine, isoleucine, proline, phenylalanine, methionine, tryptophan), amino acids having beta-branched side chains (e.g., threonine, valine, isoleucine) and amino acids having aromatic side chains (e.g., tyrosine, phenylalanine, tryptophan, histidine). It is considered that the antibody of the present invention has conservative amino acid substitutions and is still active.

In the present invention, the anti-CD3 antibody sequence may differ from the sequence provided in the present invention. For example, the amino acid sequence may (a) include the variable region separated from the constant domain of the light chain, (b) differ from the amino acid sequence according to the present invention but may not significantly affect the chemical properties of the residues (so-called "conservative substitutions"), and (c) have a homology of, for example, 80%, 85%, 90%, 91%, 92%, 93%, 94%, 95%, 96%, 97%, 98% or 99% with the amino acid sequence according to the present invention. Alternatively, the nucleic acid encoding the antibody (a) may be separated from the constant domain of the light chain, (b) may differ from the nucleic acid sequence according to the present invention without changing the encoded residues, and (c) may have a homology of 70%, 75%, 80%, 85%, 90%, 91%, 92%, 93%, 94%, 95%, 96%, 97%, 98% or 99% with the nucleic acid sequence according to the present invention.

In the present invention, the hydropathic index of the amino acid may be considered when inducing conservative changes in the amino acid sequence. The importance of the hydropathic amino acid index in imparting mutual biological functions to proteins is generally understood in the art. The relative hydropathic properties of amino acids contribute to the secondary structure of the resulting protein, which determines the interaction of the protein with other molecules, such as enzymes, substrates, receptors, DNA, antibodies, and antigens.

In the present invention, it is also known in the art that substitution of similar amino acids may be efficiently performed based on hydrophilicity. For example, the highest local average hydrophilicity of a protein, which greatly depends on the hydrophilicity of adjacent amino acids, is associated with the biological properties of the protein. It is understood that an amino acid may be substituted with another amino acid having similar hydrophilicity, and may produce a biologically or immunologically modified protein. In such a change, substitution with an amino acid having a hydrophilicity of ±2 or less is preferred, an amino acid having a hydrophilicity of ±1 or less is particularly preferred, and an amino acid having a hydrophilicity of ±0.5 or less is even more particularly preferred.

As described above, amino acid substitutions are generally based on the relative similarity of the amino acid side chain substituents, such as hydrophobicity, hydrophilicity, charge, and size. Exemplary substitutions made in consideration of the various characteristics described above are well known to those skilled in the art and include: arginine and lysine; glutamate and aspartate; serine and threonine; glutamine and asparagine; and valine, leucine, and isoleucine;

In the present invention, the anti-CD3 antibody encompasses an antigen-binding fragment thereof, which is a fragment having the function of binding to the antigen of the anti-CD3 antibody, i.e., CD3, and also includes Fab, Fab', F(ab')₂, scFv, (scFv)₂, scFv-Fc, and Fv, and the like.

Fab refers to a structure including a variable region of each of the heavy chain and the light chain, the constant region of the light chain, and the first constant domain (CH1) of the heavy chain, each having one antigen-binding site. Fab' is different from Fab in that it further includes a hinge region including at least one cysteine residue at the C-terminus of the CH1 domain of the heavy chain. F(ab')₂ is created by a disulfide bond between cysteine residues in the hinge region of Fab'. Fv (variable fragment) is the minimal antibody fragment having only a heavy-chain variable region and a light-chain variable region. Two-chain Fv (dsFv) is a fragment wherein the variable region of the heavy chain and the variable region of the light chain are linked by a non-covalent bond, and single-chain Fv (scFv) is a fragment wherein the variable region of the heavy chain and the variable region of the light chain are generally linked by a covalent bond via a peptide linker therebetween. Such antibody fragments may be obtained using proteases (*e.g.*, Fab may be obtained by restriction-cleaving the complete antibody with papain, and the F(ab')₂ fragment may be obtained by restriction-cleaving the complete antibody with pepsin), and may be prepared using genetic recombination techniques (for example, amplification using DNA encoding a heavy chain or a variable region thereof of an antibody and DNA encoding a light chain or a variable region thereof as templates by PCR (polymerase chain reaction) using a primer pair, and amplification using a combination of DNA encoding a peptide linker with a primer pair, the two ends of which are linked to the heavy chain or the variable region thereof and the light chain or a variable region thereof, respectively).

In the present invention, the first nucleic acid sequence may include the epsilon chain of an anti-CD3 antibody.

Numerous CD3 antibodies known in the art have been reported to recognize the CD3 epsilon subunit of the CD3 complex, but in fact most thereof bind to conformational epitopes and thus recognize only CD3 epsilon in the natural context of the T cell receptor (TCR). A conformational epitope is characterized by the presence of two or more non-consecutive amino acid residues that are separated in the primary sequence but come together on the molecular surface when the polypeptide folds into a native protein/antigen ([Sela, (1969) Science 166, 1365] and [Laver, (1990) Cell 61, 553-6]). The conformational epitopes bound by CD3 epsilon antibodies known in the art may be divided into two groups. In the major group, the epitope is formed by two CD3 subunits, e.g., the CD3 epsilon chain and the CD3 gamma or CD3 delta chain. For example, some studies have shown that OKT3, WT31, UCHT1, 7D6, and Leu-4, which are the most widely used CD3 epsilon monoclonal antibodies, do not bind to cells transfected with the CD3-epsilon chain alone. However, these antibodies are stained with cells doubly transfected with combinations of CD3 epsilon with either CD3 gamma or CD3 delta ([Tunnacliffe, supra]; [Law, Int. Immunol. 14 (2002), 389-400]; [Salmeron, J. Immunol. 147 (1991), 3047-52]; [Coulie, Eur. J. Immunol. 21 (1991), 1703-9]). In a second subgroup, conformational epitopes are formed within the CD3 epsilon subunit itself. A member of this group is, for example, the mAb APA 1/1, which is generated against a mutated CD3 epsilon ([Risueno, Blood 106 (2005), 601-8]). Overall, most of the CD3 epsilon antibodies disclosed in the art recognize conformational epitopes located on two or more subunits of CD3. The discrete amino acid residues forming the three-dimensional structure of these epitopes may be the CD3 epsilon subunit itself or located on the CD3 epsilon subunit and on other CD3 subunit such as CD3 gamma or CD3 delta.

Anti-CD3 monoclonal antibodies act by highly specifically recognizing the target molecule thereof. They recognize only a single site or epitope on the target CD3 molecule thereof. For example, one of the most widely used and most characteristic monoclonal antibodies specific for the CD3 complex is OKT-3. This antibody reacts with chimpanzee CD3, but not with CD3 homologs of other primates, such as macaques, or with dog CD3 ([Sandusky et al., J. Med. Primatol. 15 (1986), 441-451]). Similarly, WO 2005/118635 and WO 2007/033230 disclose human monoclonal CD3 epsilon antibodies which react with human CD3 epsilon, but not with CD3 epsilon of mouse, rat, rabbit or non-chimpanzee primates, such as rhesus macaques, cynomolgus macaques or baboon.

In the present invention, the membrane protein may include a GPI-anchoring domain of a GPI-anchoring protein or a transmembrane domain of a transmembrane protein.

As used herein, the term "membrane protein" means a protein inserted into or attached to the surface of a membrane including a lipid bilayer, and includes an "intrinsic membrane protein", which is a protein inserted into a lipid bilayer, an "extrinsic membrane protein", which is a protein attached to the surface of a lipid bilayer, or the like, and the intrinsic membrane protein is a transmembrane protein that crosses the membrane.

In the present invention, the extrinsic membrane protein is linked to a cell membrane through a covalent or non-covalent bond with a membrane protein and a lipid bilayer or transmembrane protein. A lipoprotein is formed through a covalent bond between a protein molecule and a lipid molecule, and a hydrophobic bond is formed between the lipid molecule of the lipoprotein and the lipid monolayer of the cell membrane, as a result, the protein is fixed to the surface of the cell membrane, and an example thereof is GPI (glycosylphosphatidylinositol)-anchored protein. GPI anchors a protein to the surface of a lipid bilayer by inserting a fatty acid of GPI into the lipid monolayer and covalently linking a phosphate group to a protein on the other side.

In the present invention, "GPI-anchoring protein" means a glycosylphosphatidylinositol (GPI)-anchored protein, which is covalently linked to the outer cell membrane layer and exhibits GPI-anchoring protein activity.

In the present invention, the GPI-anchoring protein may be any known GPI-anchoring protein. In some embodiments, the type of the GPI-anchoring protein includes, but is not limited to, LFA-3.

It is known that GPI-anchoring proteins are attached to the outer surface of the plasma membrane through GPI-anchoring. In a variety of organisms, including protozoa and vertebrates, the GPI-anchoring process is performed in the ER lumen by cleavage of the C-terminal carboxyl group of the protein and secondary addition of a core structure including ethanolamine phosphate, trimannosides, glucosamine, and inositol phospholipids (Udenfriend S, Kodukula K (1995) Annu Rev Biochem 64: 563-591). GPI-anchoring has been implicated in plasma membrane targeting, polarized targeting of apical plasma membrane, lipid raft assembly, plasma membrane recycling, and signal sensing and transduction (Mayor S, Riezman H (2004) Nat Rev Mol Cell Biol 5: 110-120).

In the present invention, an "intrinsic membrane protein" is a protein that penetrates the cell membrane and mainly includes three domains, namely, a "transmembrane domain" that penetrates the membrane, a "cytoplasmic domain" located in the cytoplasm, and an "extracellular domain" exposed to the outside of the cell.

In the present invention, the number of transmembrane domains varies depending on the membrane protein. Since the lipid bilayer includes highly hydrophobic lipids, most of the transmembrane domains that pass through this region include hydrophobic amino acids. Almost all transmembrane domains have an α-helix structure. In membrane proteins including multiple transmembrane domains, the transmembrane domains are disposed in a circular form to form a cylindrical structure. This cylindrical structure is used as a passage for transporting specific ions or biological substances by opening and closing in response to specific signals. The transmembrane domains of an extremely limited number of membrane proteins have in a β-sheet structure. A cylindrical structure formed by disposing several domains having β-sheet structure in a circular form is called a "β-barrel(β". A representative example of an integral membrane protein formed as a β-barrel is a porin present in the outer membrane of mitochondria.

In the present invention, the transmembrane protein may be selected from the group consisting of CD28, CD3 zeta, CD8 alpha, and CD4, but is not limited thereto.

In the present invention, the GPI-anchoring domain may include an amino acid sequence of SEQ ID NO: 13 and the transmembrane domain may include an amino acid sequence of SEQ ID NO: 17, but is not limited thereto.

In the present invention, the second nucleic acid sequence encoding the GPI-anchoring domain may include a sequence of SEQ ID NO: 14 and the second nucleic acid sequence encoding the transmembrane domain may include a sequence of SEQ ID NO: 18, but is not limited thereto.

Meanwhile, a protein that includes an amino acid sequence having a sequence homology of 80% or more, preferably a sequence homology of 90% or more, and more preferably a sequence homology of 99% with the amino acid sequence of SEQ ID NO: 13 or 17 and has the same characteristics as the membrane protein according to the present invention also falls within the scope of the membrane protein according to the present invention.

In the present invention, the scFv of the anti-CD3 antibody may be expressed on the cell membrane of an immune cell by a GPI-anchoring domain or a transmembrane domain.

In the present invention, the first nucleic acid sequence encoding the scFv of the anti-CD3 antibody and the second nucleic acid sequence encoding the membrane protein may be directly bonded or may be bonded via a linker.

In the present invention, the linker may be an IgG4 hinge (S228P), but is not limited thereto.

In the present invention, the IgG4 hinge (S228P) may include an amino acid sequence of SEQ ID NO: 19 and the sequence encoding the IgG4 hinge (S228P) may include a sequence of SEQ ID NO: 20.

The sequence according to the present invention is shown in Table 1 below.

**[Table 1]**

| | Nucleic acid or amino acid sequence | SEQ ID NO |
|---|---|---|
| Heavy chain variable region of 145-2C11 | MNSGLQLVFFVLTLKGIQGEVQLVESGGGLVQPGKSLKLSCE ASGFTFSGYGMHWVRQAPGRGLESVAYITSSSINIKYADAVK GRFTVSRDNAKNLLFLQMNILKSEDTAMYYCARFDWDKNYW GQGTMVTVSSAKTTAP | 1 |
| | ATGAACTCAGGACTCCAATTGGTTTTCTTTGTCCTCACTCTA AAAGGTATACAGGGTGAGGTGCAGCTGGTGGAGTCTGGGG GAGGCTTGGTGCAGCCTGGAAAGTCCCTGAAACTCTCCTGT GAGGCCTCTGGATTCACCTTCAGCGGCTATGGCATGCACTG GGTCCGCCAGGCTCCAGGGAGGGGGCTGGAGTCGGTCGCA TACATTACTAGTAGTAGTATTAATATCAAATATGCTGACGC TGTGAAAGGCCGGTTCACCGTCTCCAGAGACAATGCCAAG AACTTACTGTTTCTACAAATGAACATTCTCAAGTCTGAGGA CACAGCCATGTACTACTGTGCAAGATTCGACTGGGACAAAA ATTACTGGGGCCAAGGAACCATGGTCACCGTCTCCTCAGCC AAAACAACAGCCCCA | 2 |
| Light chain variable region of 145-2C11 | DIQMTQSPSSLPASLGDRVTINCQASQDISNYLNWYQQKPGKA PKLLIYYTNKLADGVPSRFSGSGSGRDSSFTISSLESEDIGSYYC QQYYNYPWTFGPGTKLEIKR | 3 |
| | GACATCCAGATGACCCAGTCTCCATCATCACTGCCTGCCTC CCTGGGAGACAGAGTCACTATCAATTGTCAGGCCAGTCAGG ACATTAGCAATTATTTAAACTGGTACCAGCAGAAACCAGGG AAAGCTCCTAAGCTCCTGATCTATTATACAAATAAATTGGC AGATGGAGTCCCATCAAGGTTCAGTGGCAGTGGTTCTGGGA GAGATTCTTCTTTCACTATCAGCAGCCTGGAATCCGAAGAT ATTGGATCTTATTACTGTCAACAGTATTATAACTATCCGTGG ACGTTCGGACCTGGCACCAAGCTGGAAATCAAACGG | 4 |
| Heavy chain variable region of OKT3 antibody | | 5 |
| | CAGGTCCAGCTGCAGCAGTCTGGGGCTGAACTGGCAAGAC CTGGGGCCTCAGTGAAGATGTCCTGCAAGGCTTCTGGCTAC ACCTTTACTAGGTACACGATGCACTGGGTAAAACAGAGGCC TGGACAGGGTCTGGAATGGATTGGATACATTAATCCTAGCC GTGGTTATACTAATTACAATCAGAAGTTCAAGGACAAGGCC ACATTGACTACAGACAAATCCTCCAGCACAGCCTACATGCA ACTGAGCAGCCTGACATCTGAGGACTCTGCAGTCTATTACT GTGCAAGATATTATGATGATCATTACTGCCTTGACTACTGG GGCCAAGGCACCACTCTCACAGTCTCCTCA | 6 |
| Light chain variable region of OKT3 antibody | QIVLTQSPAIMSASPGEKVTMTCSASSSVSYMNWYQQKSGTSP KRWIYDTSKLASGVPAHFRGSGSGTSYSLTISGMEAEDAATYY CQQWSSNPFTFGSGTKLEINR | 7 |
| | CAAATTGTTCTCACCCAGTCTCCAGCAATCATGTCTGCATCT CCAGGGGAGAAGGTCACCATGACCTGCAGTGCCAGCTCAA GTGTAAGTTACATGAACTGGTACCAGCAGAAGTCAGGCAC CTCCCCCAAAAGATGGATTTATGACACATCCAAACTGGCTT CTGGAGTCCCTGCTCACTTCAGGGGCAGTGGGTCTGGGACC TCTTACTCTCTCACAATCAGCGGCATGGAGGCTGAAGATGC TGCCACTTATTACTGCCAGCAGTGGAGTAGTAACCCATTCA CGTTCGGCTCGGGGACAAAGTTGGAAATAAACCGG | 8 |
| M-aCD3 scFv (Codon optimization) | METDTLLLWVLLLWVPGSTGDYPYDVPDYA D IQMTQSPSSLPASLGDRVTINCQASQDISNYLNWYQQKPGKAP KLLIYYTNKLADGVPSRFSGSGSGRDSSFTISSLESEDIGSYYCQ QYYNYPWTFGPGTKLEIKR EVQLVESG GGLVQPGKSLKLSCEASGFTFSGYGMHWVRQAPGRGLESVA YITSSSINIKYADAVKGRFTVSRDNAKNLLFLQMNILKSEDTA MYYCARFDWDKNYWGQGTMVTVSS ASTTCIPSSGHSRHRYALIPIPLAVITTCIVLYMNVL (Red: leader sequence Yellow: HA tag Light blue: anti-CD3 light chain variable region Dark blue: anti-CD3 heavy chain variable region Orange: LFA-3 GPI-anchoring domain) | 9 |
| | ATGGAAACCGACACACTGCTGCTGTGGGTGCTGCTTCTTTG GGTGCCCGGAAGCACAGGCGACTACCCTTACGATGTGCCTG ACTATGCT GA TATCCAGATGACACAGAGCCCCAGCAGCCTGCCTGCTTCTC TGGGAGACAGAGTGACCATCAACTGCCAGGCCAGCCAGGA CATCAGCAACTACCTGAACTGGTATCAGCAGAAGCCCGGC AAGGCCCCTAAGCTGCTGATCTACTACACCAACAAGCTGGC CGACGGCGTGCCCAGCAGATTTTCTGGTTCTGGCAGCGGCA GAGACAGCAGCTTCACAATCAGCTCCCTGGAAAGCGAGGA CATCGGCAGCTACTACTGCCAGCAGTACTACAACTACCCCT GGACATTCGGCCCTGGCACCAAGCTGGAAATCAAGAGA GAAGTTCAGCTGGTTGAATCAGGTGGCGGCCTGGTGC AGCCTGGCAAGTCTCTGAAGCTGTCTTGCGAGGCCAGCGGC TTCACCTTTTCTGGCTACGGAATGCACTGGGTCCGACAGGC ACCTGGCAGAGGACTGGAATCTGTGGCCTACATCACCAGCA GCAGCATCAACATTAAGTACGCCGACGCCGTGAAGGGCAG ATTCACCGTGTCCAGAGACAACGCCAAGAACCTGCTGTTCC TGCAGATGAACATCCTGAAGTCCGAGGACACCGCCATGTAC TACTGTGCCAGATTCGACTGGGACAAGAACTACTGGGGCCA GGGCACCATGGTCACAGTTTCTAGT GCCAGC ACCACATGCATCCCTTCTAGCGGACACAGCAGACACAGATA CGCTCTGATCCCCATTCCTCTGGCCGTGATCACCACCTGTAT CGTGCTGTACATGAACGTGCTGTGA (Red: leader sequence Yellow: HA tag Light blue: anti-CD3 light chain variable region Dark blue: anti-CD3 heavy chain variable region Orange: LFA-3 GPI-anchoring domain) | 10 |
| CDS(coding sequence) of LFA-3 | MVAGSDAGRALGVLSVVCLLHCFGFISCFSQQIYGVVYGNVT FHVPSNVPLKEVLWKKQKDKVAELENSEFRAFSSFKNRVYLD TVSGSLTIYNLTSSDEDEYEMESPNITDTMKFFLYVLESLPSPT LTCALTNGSIEVQCMIPEHYNSHRGLIMYSWDCPMEQCKRNS TSIYFKMENDLPQKIQCTLSNPLFNTTSSIILTTCIPSSGHSRHRY ALIPIPLAVITTCIVLYMNGILKCDRKPDRTNSN | 11 |
| | atggttgctgggagcgacgcggggcgggccctgggggtcctcagcgtggtctgcctgctgcact gctttggtttcatcagctgtttttcccaacaaatatatggtgttgtgtatgggaatgtaactttccatgta ccaagcaatgtgcctttaaaagaggtcctatggaaaaaacaaaaggataaagttgcagaactgga aaattctgaattcagagctttctcatcttttaaaaatagggtttatttagacactgtgtcaggtagcctc actatctacaacttaacatcatcagatgaagatgagtatgaaatggaatcgccaaatattactgatac catgaagttctttctttatgtgcttgagtctcttccatctcccacactaacttgtgcattgactaatggaa gcattgaagtccaatgcatgataccagagcattacaacagccatcgaggacttataatgtactcatg ggattgtcctatggagcaatgtaaacgtaactcaaccagtatatattttaagatggaaaatgatcttc cacaaaaaatacagtgtactcttagcaatccattatttaatacaacatcatcaatcattttgacaacct gtatcccaagcagcggtcattcaagacacagatatgcacttatacccataccattagcagtaattac aacatgtattgtgctgtatatgaatggtattctgaaatgtgacagaaaaccagacagaaccaactcc aattga | 12 |
| GPI-anchoring domain of LFA-3 | TTCIPSSGHSRHRYALIPIPLAVITTCIVLYMN | 13 |
| | acaacctgtatcccaagcagcggtcattcaagacacagatatgcacttatacccataccattagca gtaattacaacatgtattgtgctgtatatgaatggtatt | 14 |
| CDS(coding sequence) of CD28 | MLRLLLALNLFPSIQVTGNKILVKQSPMLVAYDNAVNLSCKY SYNLFSREFRASLHKGLDSAVEVCVVYGNYSQQLQVYSKTGF NCDGKLGNESVTFYLQNLYVNQTDIYFCKIEVMYPPPYLDNE KSNGTIIHVKGKHLCPSPLFPGPSKPFWVLVVVGGVLACYSLL VTVAFIIFWVRSKRSRLLHSDYMNMTPRRPGPTRKHYQPYAPP RDFAAYRS | 15 |
| | atgctcaggctgctcttggctctcaacttattcccttcaattcaagtaacaggaaacaagattttggtg aagcagtcgcccatgcttgtagcgtacgacaatgcggtcaaccttagctgcaagtattcctacaat ctcttctcaagggagttccgggcatcccttcacaaaggactggatagtgctgtggaagtctgtgttg tatatgggaattactcccagcagcttcaggtttactcaaaaacggggttcaactgtgatgggaaatt gggcaatgaatcagtgacattctacctccagaatttgtatgttaaccaaacagatatttacttctgcaa aattgaagttatgtatcctcctccttacctagacaatgagaagagcaatggaaccattatccatgtga aagggaaacacctttgtccaagtcccctatttcccggaccttctaagcccttttgggtgctggtggt ggttggtggagtcctggcttgctatagcttgctagtaacagtggcctttattattttctgggtgaggag taagaggagcaggctcctgcacagtgactacatgaacatgactccccgccgccccgggcccac ccgcaagcattaccagccctatgccccaccacgcgacttcgcagcctatcgctcctga | 16 |
| Transmembrane domain of CD28 | FWVLVVVGGVLACYSLLVTVAFIIFWV | 17 |
| | ttttgggtgctggtggtggttggtggagtcctggcttgctatagcttgctagtaacagtggcctttatt attttctgggtg | 18 |
| IgG4 hinge(S228P) | ESKYGPPCPPCP | 19 |
| | GAGTCTAAGTACGGACCTCCTTGTCCTCCATGTCCT | 20 |

In the present invention, the first nucleic acid sequence encoding the scFv of the anti-CD3 antibody and the second nucleic acid sequence encoding the membrane protein are introduced by transfection.

In the present invention, the transfection includes, but is not limited to, cationic liposome method, lithium acetate-DMSO, lipid-mediated transfection, calcium phosphate precipitation, electroporation, lipofection, PEI (polyethyleneimine)-mediated transfection, DEAE-dextran-mediated transfection, and nanoparticle-mediated nucleic acid delivery.

In the present invention, the transfection is preferably performed using a reagent capable of encapsulating DNA plasmid containing a gene construct including the first nucleic acid sequence and the second nucleic acid sequence in a liposome and delivering the result into cells, and a transfection reagent generally used in the art, for example, Lipofectamine MessengerMAX^{™} reagent (Therfmo Fisher), Lipofectamine 2000 reagent (Invitrogen), and Lipofectamine 3000 reagent (Invitrogen) may be used.

In the present invention, the first nucleic acid sequence and the second nucleic acid sequence are introduced by a vector including the first nucleic acid sequence encoding the scFv of the anti-CD3 antibody and the second nucleic acid sequence encoding the membrane protein.

In the present invention, the vector may be a known vector such as a plasmid vector, cosmid vector, or bacteriophage vector and the vector may be easily prepared by those skilled in the art depending on any known method using DNA recombination technology.

In the present invention, the immune cell may be a macrophage.

In the present invention, the immune cell means a cell involved in an immune response in the body, any immune cell may be used without particular limitation as long as it is known as an immune cell in the art, especially known as an immune cell present in the human body, and examples thereof include monocytes, macrophages, neutrophils, eosinophils, basophils, dendritic cells, natural killer cells, megakaryocytes, T cells, and B cells. Preferably, the immune cell may mean a monocyte, a macrophage, or a neutrophil. More preferably, a macrophage.

As used herein, the term "immune response" is the same as understood in the art, generally refers to a biological response of a vertebrate to a foreign agent or an abnormality (e.g., a cancer cell), and this response protects the organism against these agents and diseases caused thereby. The immune response is mediated by the action of one or more cells of the immune system (e.g., T lymphocytes, B lymphocytes, natural killer (NK) cells, macrophages, eosinophils, mast cells, dendritic cells, or neutrophils) and soluble macromolecules produced by any of these cells, or by the action of the liver (including antibodies, cytokines, and complement) to selectively target, bind, damage, destroy, and/or remove a normal human cell or tissue from the body of the vertebrate in an invading pathogen, a cell or tissue infected with a pathogen, a cancerous or other abnormal cell or autoimmune or pathological inflammation. An immune response includes, for example, activation or inhibition of T cells, such as effector T cells, Th cells, CD4+ cells, CD8⁺ T cells, or Treg cells, or activation or inhibition of any other cells of the immune system, such as NK cells.

In another aspect, the present invention is directed to a pharmaceutical composition for treating cancer containing the recombinant immune cell and a pharmaceutically acceptable carrier.

In the present invention, the recombinant immune cells may be directly injected into a tumor.

As used herein, the terms "cancer" and "tumor", which are used interchangeably, refer to or mean the physiological state of mammals, typically characterized by unregulated cell growth/proliferation.

The cancer or carcinoma that may be treated with the composition of the present invention is not particularly limited and includes both solid cancers and hematological cancers. For example, such a cancer may be selected from the group consisting of squamous cell carcinoma, small cell lung cancer, non-small cell lung cancer, adenocarcinoma of the lung, squamous cell carcinoma of the lung, peritoneal cancer, skin cancer, melanoma, rectal cancer, anal cancer, esophageal cancer, small intestine cancer, endocrine cancer, parathyroid cancer, adrenal cancer, soft tissue sarcoma, urethral cancer, chronic or acute leukemia, lymphoma, hepatocellular carcinoma, gastrointestinal cancer, pancreatic cancer, glioblastoma, cervical cancer, ovarian cancer, liver cancer, bladder cancer, hepatoma, breast cancer, colon cancer, large intestine cancer, endometrial or uterine cancer, salivary gland cancer, kidney cancer, prostate cancer, vulvar cancer, thyroid cancer, head and neck cancer, brain cancer, and osteosarcoma, but is not limited thereto.

As used herein, the term "pharmaceutically acceptable carrier" refers to a substance that may be added to an active ingredient to help formulate or stabilize a preparation, and does not cause significant harmful toxic effects to the patient.

The carrier refers to a carrier or diluent that does not stimulate the patient and does not inhibit the biological activity and properties of the administered compound. Pharmaceutically acceptable carriers for compositions formulated into liquid solutions are sterilized and biocompatible, and examples thereof include saline, sterile water, buffered saline, albumin injection solutions, dextrose solutions, maltodextrin solutions, glycerol, and mixtures thereof. If necessary, other conventional additives such as antioxidants, buffers and bacteriostatic agents may be added. In addition, injectable solutions such as aqueous solutions, suspensions and emulsions, pills, capsules, granules, or tablets may be formulated by further adding diluents, dispersants, surfactants, binders and lubricants. Other carriers are disclosed, for example, in Remington's Pharmaceutical Sciences (E. W. Martin). Such compositions may contain a therapeutically effective amount of one or more recombinant immune cells according to the present invention.

Pharmaceutically acceptable carriers include sterile aqueous solutions or dispersions and sterile powders for the extemporaneous preparation of sterile injectable solutions or dispersions. The use of such media and agents for pharmaceutical active substances is well known in the art. The composition is preferably formulated for parenteral injection. The composition may be formulated as a solution, microemulsion, liposome, or other ordered structures suitable for high drug concentrations. The carrier may be, for example, a solvent or dispersion medium containing water, ethanol, a polyol (such as glycerol, propylene glycol and liquid polyethylene glycol) and a suitable mixture thereof. In some cases, the composition may contain isotonic agents, for example, sugars, polyalcohols such as mannitol, sorbitol or sodium chloride. Sterile injectable solutions may be prepared by incorporating a required amount of the active compound in an appropriate solvent, optionally along with one of the ingredients described above or a combination thereof, followed by sterile microfiltration. In general, dispersions are prepared by incorporating the active compound into a basic dispersion medium and a sterile vehicle containing other necessary ingredients selected from among those described above. In sterile powders for the preparation of sterile injectable solutions, some preparation methods involve vacuum drying and freeze-drying (lyophilizing) to produce powders of the active ingredient and any additional desired ingredients from a pre-sterilized and filtered solution thereof.

The dosage of the pharmaceutical composition of the present invention is not particularly limited, but depends on a variety of factors including health conditions and the body weight of patient, the severity of disease, the type of drug, the administration route and the administration period. The pharmaceutical composition according to the present invention may be administered in a single dose or multiple doses daily to mammals including rats, mice, domestic animals, humans and the like, via a typically acceptable route, for example, orally, intraperitoneally, intravenously, intramuscularly, subcutaneously, intradermally, orally, topically, intranasally, intrapulmonarally or rectally, but is not limited thereto.

As used herein, the term "therapeutically effective amount" refers to an amount of recombinant immune cells required to cause a measurable benefit *in vivo* in a patient in need of treatment. The precise amount will vary depending on numerous factors including, but not limited to, the components and physical characteristics of the therapeutic composition, the intended patient population, considerations of each patient, and the like, and may be readily determined by those skilled in the art. Taking all of these factors into consideration, it is important to administer the minimum amount sufficient to achieve maximum effect without adverse effects and this dosage may be readily determined by those skilled in the art.

In another aspect, the present invention is directed to a method for treating cancer and inhibiting cancer growth including administering to a patient a pharmaceutical composition containing the recombinant immune cell as an active ingredient.

When the pharmaceutical composition containing the recombinant immune cell according to the present invention as an active ingredient is administered, a first nucleic acid sequence encoding scFv of an anti-CD3 antibody introduced into a recombinant immune cell is expressed on the cell membrane by expression of a second nucleic acid sequence encoding a membrane protein introduced into the recombinant immune cell. At this time, polyclonal proliferation of tumor-infiltrating T cells is induced in the tumor through the scFv of the anti-CD3 antibody expressed on the cell membrane and various costimulatory molecules of macrophages, and tumor growth is inhibited or delayed.

Hereinafter, the present invention will be described in more detail with reference to examples. However, it is obvious to those skilled in the art that these examples are provided only for illustration of the present invention and should not be construed as limiting the scope of the present invention.

### Production Example 1. Production of synthetic gene for cell membrane-expressed anti-CD3 epsilon scFv (M-aCD3εscFv)

A sequence encoding a variable region of an anti-CD3 epsilon antibody, 145-2C11, was linked to a GPI-anchoring coding sequence of a GPI-anchoring protein, LFA-3, and a sequence encoding a G₄S linker was linked to a sequence encoding an IgG4 hinge (S228P). Then, a sequence encoding an HA-tag (human influenza hemagglutinin-tag) for detection, separation, and purification of a target protein was linked to produce an anti-CD3 epsilon scFv (M-aCD3εscFv) gene that may be expressed on the cell membrane surface through GPI-anchoring (FIG. 1, top). The DNA fragment encoding the corresponding synthetic gene (Macrogen, Korea) was synthesized and was then subcloned into the pMRNAxp vector (System Biosciences), which was used for mRNA *in vitro* transcription.

### Production Example 2. Production of membrane-expressing EGFP (M-EGFP) synthetic gene

For expression of a control mRNA that does not contain the target gene, a sequence encoding EGFP, which is a green tubular protein, was linked to a GPI-anchoring encoding sequence of LFA-3, which is a GPI-anchoring protein, and a sequence encoding a G₄S linker was linked to a sequence encoding an IgG4 hinge (S228P). In addition, a sequence encoding HA-tag (human influenza hemagglutinin-tag) for detection, separation, and purification of the target protein was linked to produce an EGFP (M-EGFP) gene that may be expressed on the cell membrane surface through GPI-anchoring (FIG. 1, bottom). The DNA fragment encoding the synthetic gene (Macrogen, Korea) was synthesized and subcloned into the pMRNA^{xp} vector, which was used for mRNA in vitro transcription.

### Example 1. Expression of M-aCD3εscFv of proinflammatory bone marrow-derived macrophages on cell membrane

### 1.1. Production of proinflammatory bone marrow-derived macrophages (M1 BMDM)

Bone marrow cells isolated from the femur and tibia of 7-week-old female C57BL/6N mice were cultured in a medium containing 20 ng/ml murine M-CSF (Peprotech). 6 days of after culture, macrophages differentiated from mouse bone marrow cells (bone marrow-derived macrophages, BMDM) were obtained. The differentiated macrophages were treated with 40 ng/ml murine interferon-γ (IFN-γ), and after 24 hours of treatment, BMDM were polarized into proinflammatory bone marrow-derived macrophages (M1 BMDM).

### 1.2. Validation of cell membrane expression of M-aCD3εscFv

M-aCD3εscFv mRNA was produced by *in vitro* transcription from the pMRNAxp vector containing the M-aCD3εscFv gene DNA fragment prepared in Production Example 1. mRNA was synthesized using HiScribe T7 high yield RNA synthesis kit (NEB) and UTP of mRNA was completely replaced with N¹-methylpseudo-UTP (Jena Bioscience). In addition, the synthesized mRNA was used in the experiment after capping using vaccinia capping system (NEB) and mRNA cap 2'-O-methyltransferase (NEB) and tailing using *E. coli* poly (A) polymerase (NEB).

0.5 µg of the produced M-aCD3εscFv mRNA was transfected into 1X10⁵ M1 BMDMs by treatment with 1 µg of Lipofectamine^{™} MessengerMAX^{™} reagent, as a transfection reagent. After transfection, the cell membrane expression of M-aCD3εscFv was validated over time using flow cytometry.

The result shows that M-aCD3εscFv expression was maintained on the cell membrane for about 48 hours (FIG. 2).

### Example 2. Induction of polyclonal proliferation of T cells in vitro by TIL-ex MΦ

### 2.1. Production of macrophages (TIL-ex MΦ and M-EGFP-M1) and interaction with splenic T cells

The M-aCD3εscFv gene DNA fragment produced in Production Example 1 was produced into M-aCD3εscFv mRNA using the same method as in Example 1, and then transfected into M1 BMDM to produce macrophages (TIL-ex MΦ).

Meanwhile, for production of control macrophages that do not contain the target gene, the M-EGFP gene produced in Production Example 2 was produced as M-EGFP mRNA using the same method as in Example 1 and then transfected into M1 BMDM to produce macrophages (M-EGFP-M1).

Splenic T cells were isolated from the spleen of 7-week-old female C57BL/6N mice using the mouse Pan T Cell Isolation Kit II (Miltenyi Biotec). The isolated 5X10⁴ splenic T cells were co-cultured with 5X10⁴ TIL-ex MΦ. 24 hours after co-culture, the interaction between TIL-ex MΦ and splenic T cells was validated by immunocytochemistry and confocal imaging (FIG. 3A).

### 2.2. Induction of polyclonal proliferation of T cells in vitro with TIL-ex MΦ and M-EGFP-M1

Splenic T cells were stained with CellTrace^{™}Violet (CTV) and 1x10⁵ CTV-T cells were co-cultured with 2x10⁴ M-EGFP-M1 or TIL-ex MΦ in 10% FBS RPMI medium containing 50 µM β-mercaptoethanol (Sigma) using a round bottom 96-well plate (SPL). 3 days after co-culture, the degree of T cell proliferation was validated by microscopy and flow cytometry. The result showed that TIL-ex MΦ expressing M-aCD3εscFv induced polyclonal proliferation of T cells more than M-EGFP-M1 not expressing M-aCD3εscFv (FIGs. 3B to 3D).

### Example 3. Validation of induction of proliferation of tumor-infiltrating T cells by TIL-ex MΦ in vivo

### 3.1. Preparation of B16F10 mouse melanoma model and interaction of TIL and macrophage

A B16F10 mouse melanoma model was produced by subcutaneously injecting 5x10⁵ B16F10 tumor cells into the right flank of 7-week-old female C57BL/6N mice. 8 days after injection of B16F10 tumor cells, 2x10⁶ TIL-ex MΦ or M-EGFP-M1 were injected intratumorally. One day after intratumoral injection, tumors were harvested and the interaction between T cells (TILs) and macrophages infiltrating the tumor was validated by immunohistochemistry and confocal imaging (FIG. 4A).

### 3.2. Induction of in vivo proliferation of tumor-infiltrating T cells by TIL-ex MΦ and M-EGFP-M1

3 days after intratumoral injection, tumors were harvested and the number of TILs was measured through flow cytometry to validate the proliferation of TILs. The result showed that the number of CD8⁺ T cells and CD4⁺ T cells significantly increased in the TIL-ex MΦ-treated group compared to the PBS control group and the M-EGFP-M1-treated group, and in particular, the number of CD8⁺ T cells significantly increased (FIGs. 4B and 4C).

### 3.3. Proportion of TIL-ex MΦ and M-EGFP-M1 Ki-67⁺ cell and Foxp3⁺ T reg

The proportion of Ki-67⁺ cells, which are a proliferative cell marker, was validated. The result showed that Ki-67⁺ CD8⁺ T cells significantly increased in the TIL-ex MΦ administration group compared to the PBS control group and M-EGFP-M1 administration group, and Ki-67⁺ CD4⁺ T cells significantly increased in the TIL-ex MΦ administration group compared to the PBS control group (FIGs. 4D and 4E).

Meanwhile, whether or not TIL-ex MΦ administration also induced the proliferation of anti-inflammatory T_{reg} was determined. Foxp3⁺ T_{reg} decreased in the TIL-ex MΦ administration group compared to the PBS control group and M-EGFP-M1 administration group (FIG. 4F and 4H).

That is, TIL-ex MΦ not only induces the proliferation of CD8⁺ TIL when injected intratumorally, but also reduces the proportion of T_{reg}, to regulate the anti-inflammatory tumor microenvironment into an inflammatory one.

### 3.4. Antigen-specific tumor cell killing ability of intratumorally proliferated TIL

A B16F10-OVA mouse melanoma model was created by subcutaneously injecting 5x10⁵ B16F10-OVA tumor cells into the right flank of 7-week-old female C57BL/6N mice. 2x10⁶ TIL-ex MΦ or M-EGFP-M1 was injected into the B16F10-OVA tumor. 3 days after injection, intratumoral TILs were isolated through MACS (magnetic associated cell separation) using CD90.2 microbeads (Miltenyi Biotec), and the isolated TILs were co-cultured with EL4 or E.G7-OVA cancer cell lines treated with 100 ng/ml of IFN-γ for 48 hours. 12 hours after co-culture, viable tumor cells were assayed by flow cytometry to validate OVA-specific tumor cell death. The result showed that both TILs isolated from tumors of the TIL-ex MΦ administration group and the M-EGFP-M1 administration group normally exhibited OVA-specific cytotoxicity (FIG. 4H).

### Example 4. Anti-tumor effect of TIL-ex MΦ

### 4.1. Antitumor effect of TIL-ex MΦ in B16F10 mouse melanoma model

B16F10 mouse melanoma model was produced by subcutaneously injecting 2x10⁵ B16F10 tumor cells into the right flank of 7-week-old female C57BL/6N mice. 9 days after tumor cell injection, 3x10⁶ TIL-ex MΦ or M-EGFP-M1 were injected intratumorally and tumor volumes were measured at 2-day intervals. The result showed that tumor growth was significantly inhibited or delayed in the TIL-ex MΦ group compared to the M-EGFP-M1 group without any change in body weight (FIGs. 5A and 5B).

### 4.2. Anti-tumor effect of TIL-ex MΦ in MC38 mouse colon cancer model

A MC38 mouse colon cancer model was produced by subcutaneously injecting 5x10⁵ MC38 tumor cells into the right flank of 7-week-old female C57BL/6N mice. 8 days after tumor cell injection, 2x10⁶ TIL-ex MΦ or M-EGFP-M1 were injected intratumorally and tumor volumes were measured at 2-day intervals. The result showed that tumor growth was significantly inhibited or delayed in the TIL-ex MΦ group compared to the M-EGFP-M1 group without any change in body weight (FIGs. 5C and 5D).

Although the preferred embodiments of the present invention have been disclosed for illustrative purposes, those skilled in the art will appreciate that various modifications, additions and substitutions are possible, without departing from the scope and spirit of the invention as disclosed in the accompanying claims.

### [Industrial Applicability]

When the immune cell of the present invention is injected into a tumor, it induces the expression of an anti-CD3 single chain variable fragment (scFv) gene containing an epsilon chain of an anti-CD3 antibody on the cell membrane surface through the GPI-anchoring domain of a GPI-anchoring protein or the transmembrane domain of a transmembrane protein. In addition, the immune cell of the present invention induces polyclonal proliferation of tumor-infiltrating T cells *in vitro.* In addition, the immune cell of the present invention induces polyclonal proliferation of tumor-infiltrating T cells in a tumor. In addition, the immune cell of the present invention delays or suppresses tumor growth, thus exhibiting an excellent anticancer effect.

In addition, although information on cancer cell-specific antigens is not known in advance, which is a major limitation of cancer treatment, particularly solid cancer treatment, the immune cell of the present invention can provide effective anticancer immunotherapy when injected into a tumor.

Although the preferred embodiments of the present invention have been disclosed for illustrative purposes, those skilled in the art will appreciate that various modifications, additions and substitutions are possible, without departing from the scope and spirit of the invention as disclosed in the accompanying claims.

### [Sequence Listing Free Text]

An electronic file is attached.

## Claims

1. A recombinant immune cell into which (i) a first nucleic acid sequence encoding a single chain variable fragment (scFv) of an anti-CD3 antibody and (ii) a second nucleic acid sequence encoding a membrane protein are introduced.

2. The recombinant immune cell according to claim 1, wherein the scFv of the anti-CD3 antibody comprises:
a heavy chain variable region amino acid sequence of SEQ ID NO: 1 or SEQ ID NO: 5; and
a light chain variable region amino acid sequence of SEQ ID NO: 3 or SEQ ID NO: 7.

3. The recombinant immune cell according to claim 1, wherein the first nucleic acid sequence comprises:
a sequence of SEQ ID NO: 2 or SEQ ID NO: 6; and
a sequence of SEQ ID NO: 4 or SEQ ID NO: 8.

4. The recombinant immune cell according to claim 1, wherein the scFv of the anti-CD3 antibody comprises an amino acid sequence of SEQ ID NO: 9.

5. The recombinant immune cell according to claim 1, wherein the first nucleic acid sequence comprises a sequence of SEQ ID NO: 10.

6. The recombinant immune cell according to claim 1, wherein the first nucleic acid sequence is an epsilon chain of the anti-CD3 antibody.

7. The recombinant immune cell according to claim 1, wherein the membrane protein comprises a GPI-anchoring domain of a GPI-anchoring protein or a transmembrane domain of a transmembrane protein.

8. The recombinant immune cell according to claim 7, wherein the GPI-anchoring domain comprises an amino acid sequence of SEQ ID NO: 13.

9. The recombinant immune cell according to claim 7, wherein the transmembrane protein is selected from the group consisting of CD28, CD3 zeta, CD8 alpha, and CD4.

10. The recombinant immune cell according to claim 7, wherein the transmembrane domain comprises an amino acid sequence of SEQ ID NO: 17.

11. The recombinant immune cell according to claim 1, wherein the scFv of the anti-CD3 antibody is expressed on a cell membrane of the immune cell by the GPI-anchoring domain or the transmembrane domain.

12. The recombinant immune cell according to claim 1, wherein the first nucleic acid sequence and the second nucleic acid sequence are introduced by transfection.

13. The recombinant immune cell according to claim 1, wherein the first nucleic acid sequence and the second nucleic acid sequence are introduced by a vector containing the first nucleic acid sequence and the second nucleic acid sequence.

14. The recombinant immune cell according to claim 1, wherein the immune cell is a macrophage.

15. A pharmaceutical composition for treating cancer comprising:
the recombinant immune cell according to any one of claims 1 to 14; and
a pharmaceutically acceptable carrier.

16. The pharmaceutical composition according to claim 15, wherein the recombinant immune cell is directly injected into a tumor.

17. The pharmaceutical composition according to claim 15, wherein the cancer is a solid tumor.
